# EUROPEAN PATENT SPECIFICATION

(11) **EP 1 788 009 B1**
(45) Date of publication and mention of the grant of the patent: **24.03.2010**
(21) Application number: 06124168.3
(22) Date of filing: 15.11.2006
(51) Int. Cl.: C08G 18/67, C08F 290/06, B29C 39/00, H04R 25/00

(54) **Process for the solid freeform fabrication of hearing aids using curable acrylate oligomers**
Verfahren zur Herstellung von Hörhilfe Geräten mit härtenden Acrylat-Oligomeren
Procedure pour la manufacture de prothèses auditives avec oligomères acryliques

(30) Priority: 16.11.2005 US 280039
(43) Date of publication of application: 23.05.2007
(73) Proprietor: Henkel Corporation, Rocky Hill, CT 06067 (US)
(72) Inventor: Litke, Alan Edward, Waterbury, CT 06705 (US); Kadziela, Vic, Rocky Hill, CT 06067 (US)
(74) Representative: Lane, Cathal Michael

(56) References cited:
- WO-A-2004/052957
- WO-A2-96/15179
- WO-A2-03/072623
- DE-A1- 3 940 898
- US-A- 5 763 503
- HA C-H ET AL: "PROPERTIES OF UV-CURABLE POLYURETHANE ACRYLATES USING NONYELLOWING POLYISOCYANATE FOR FLOOR COATING" JOURNAL OF APPLIED POLYMER SCIENCE, JOHN WILEY AND SONS INC. NEW YORK, US, vol. 63, no. 7, 14 November 1996 (1996-11-14), pages 1011-1021, XP000643285 ISSN: 0021-8995

## Description

The present invention relates to a method for producing high strength in-the-ear products using solid freeform fabrication ("SFF") techniques. In particular, the present invention relates to a method for the production of hearing aid components using stereolithography.

Hearing aids need to be durable, comfortable and reliable. Otherwise, end users may oftentimes resist their use. Durability focuses on the integrity of the material, long lasting, and integrity of the bonding of the components. Comfortability implies that the device is soft when placed in the ear canal. Reliability implies superior acoustic quality throughout the life of the device, which requires adequate sealing within the ear canal. The challenges to satisfy the comfort and reliability objectives are due to the dynamic nature of the ear canal, and the geometric alterations of the ear canal due to natural anatomical movement. The dynamic nature of the ear canal varies from person to person, and even the anatomical shape varies from ear to ear of the same person. The canal shape is geometrically altered by motion from the head and the mandible, usually causing elliptical elongation. These differences in canal shape and changes due to body movement make it difficult to achieve a comfortable and true acoustic seal.

Challenges in meeting comfort as well as durability are due to the nature of the ear canal and materials able to use. In the past, hearing aids were made from hard acrylic materials which have proven to be durable but uncomfortable. And when the device was displaced by motion, a leakage of sound pressure occurred. Attempts were made to use rubber instead of the hard acrylic materials, such as in U.S. Patent No. 3,527,901 to Geib. Rubber is softer and more resilient than hard acrylic but it is not very comfortable and still lacks a true acoustic seal upon motion.

Attempt to use soft vinyl materials have also not been entirely successful in meeting the aforementioned characteristics. Although vinyl may be softer than rubber and offers a better acoustic seal, soft vinyl lacks durability, and in fact, after a relatively short period of time it shrinks, turns yellow and becomes hard or brittle. It is recommended in the hearing aid industry to replace vinyl components for behind-the-ear ear molds at least annually.

Silicone materials have also been used as the housing material, such as disclosed in U.S. Patent No. 6,022,311 to Juneau et al. The '311 patent discloses a two layer silicone housing bonded with an adhesive to the plastic faceplate of the device. Although silicone has a longer wear life than vinyl materials, it lacks strong bonding properties to the plastics commonly used in hearing aid instrumentality.

Polyurethanes have in the past been used for hearing aid components. For example, U.S. Patent No. 5,763,503 to Cowperthwaite et al. discloses a housing for an in-the-ear hearing aid made from a solid and stiff polyurethane, polyesters or polyether to support the instrumentality.

Alternatively, instead of focusing on the housing material, attempts have been made to supply an attachment to the housing such as a covering or sleeve. This preserves the durability of the original housing material, while adding a comfort factor. For instance, U.S. Patent No. 4,870,688 to Voroba et al. discloses a soft, resilient covering which is affixed to the rigid bonding of the ear shell. U.S. Patent No. 5,002,151 to Oliveira et al. discloses a disposable sleeve made of a soft polyurethane retarded recovery foam attached to the ear piece by mating of screw threads on the sleeve and the ear piece. Unfortunately, a sleeve concept would lack durability and require continual replacement.

Further, hearing aid components, such as hearing aid housings, typically have been prepared by molding techniques. In general, a translucent mold representing the area of application in the individual's ear is formed. The composition is poured into the mold and cured therein to form the hearing aid component.

Therefore, there is a need for high-strength hearing aid components, which are durable and provide superior acoustics.

The present invention provides high-strength hearing aid components prepared by SFF techniques, which accurately use digital representations of the of application in the individual's ear to additively form the three-dimensional hearing aid component. An added advantage is that the three-dimensional data may be stored for future use, such as, for example, hearing aid repairs.

In some embodiments, there is provided a method of making a hearing aid component including the steps of: (a) providing data of the three-dimensional size and shape of a region in an individual's ear, wherein the region corresponds to the hearing aid component to be made; (b) providing a first amount of a curable composition including: (i) at least one acrylate oligomer selected from urethane acrylate oligomers, epoxy acrylate oligomers, metallic acrylate oligomers and combinations thereof; (ii) at least one reactive diluent selected from ethoxylated bisphenol A dimethacrylate, propoxylated neopentyl glycol diacrylate, dipentaerythritol monohydroxy pentaacrylate, isobornyl acrylate and combinations thereof; and (iii) a cure system including at least one photoinitiator; (c) exposing the composition to photo-radiation for a time and intensity sufficient to cure a layer on the surface of the composition which corresponds to a cross-section of the hearing aid component; (d) providing a second amount of a curable composition; (e) applying the second amount to the cured layer to form a new layer and exposing the new layer to photo-radiation for a time and intensity sufficent to cure the new layer; and (f) repeating steps (d) and (e) until the hearing aid component is formed.

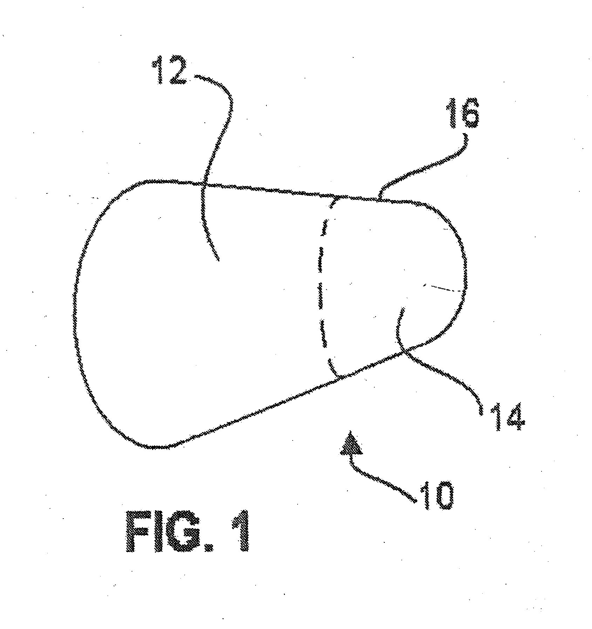

Fig. 1 shows a perspective view of a hearing aid housing attached to a tip in accordance with an embodiment of the present intention.

As shown in Fig. 1, a hearing aid housing 12 (also commonly referred to as a shell) of hearing aid assembly 10 is constructed to fit within the outer ear of the wearer. Adhesively mated to the housing 12 is a tip 14 which projects through the concha and into the auditory canal area. The outer walk 16 of tip 14 forms a seal at the opening and in the auditory canal.

The hearing aid housing 12 typically contains amplifier means, volume adjustment control and battery access door, all of which are not shown. In operation, the amplifier means for receiving and amplifying unamplified sound is connected to a sound tube adapted for conveying sound from the amplifier means to the end of the tube inside the ear canal. The tip 14 encloses the tube for conveying sound and may contain other ports for various uses such as vent apertures. The end of the sound tube is positioned to deliver sound energy generally along the axis of the ear canal when inserted.

To provide a comfortable fit and an acoustic seal in the wearer's ear, hearing aid housing 12 is formed of a curable composition, which exhibits high strength yet is sufficiently deformable to provide an acoustic seal and be comfortable to the user. The sealing and comfort properties are important for commercial viability. Additionally, tip 14 may be mated to the hearing aid housing 12. The tip 14 may be formed of the same curable composition as the hearing aid housing or alternatively the curable compositions disclosed in Applicants' U.S. Patent No. 6,829,362, entitled "Soft Molding Compound," or Applicants' U.S. Patent Application No. 10/922,458, entitled "Deformable Soft Molding Compositions" and filed on August 20, 2004, both of which are incorporated herein by reference in their entirety.

The inventive curable compositions of the present invention exhibit high strength when cured allowing formation of hearing aid components using SFF techniques. The curable compositions include the reaction product of at least one acrylate oligomer and at least one reactive diluent, designed to provide a Shore D hardness of at least about 85, as well as high tensile modulus and flexural modulus. Desirably, the tensile modulus and flexural modulus both are at least about 300,000 psi. Curing is initiated by a cure system, which may include a photoinitiator, as well as other optional components.

### Acrylate Oligomers

The curable compositions of the present invention contain at least one acrylate oligomer. The acrylate oligomer may be a urethane acrylate oligomer, an epoxy acrylate oligomer, a metallic acrylate oligomer or combinations thereof.

The urethane acrylate oligomer may be selected from a variety of materials, most desirably aliphatic urethane acrylates. Useful urethane acrylates include di- or tri-functionalized aliphatic urethane acrylates, which are capable of cross-linking during cure.

Representative commercially available urethane acrylate oligomers useful in the compositions of the present invention include EBECRYL 8402 (available from UCB Group), PHOTOMER 6008, 6019 and 6210 (available from Cognis, Corp.) and BR-582 (available from Bomar Specialties Co.).

EBECRYL 8402 is a low viscosity aliphatic urethane diacrylate. The viscosity of EBECRYL 8402 (neat) is about 500 cps at 65.5°C. EBECRYL 8402 typically exhibits the following properties: tensile strength of about 3300 psi and tensile elongation percent of about 90.

PHOTOMER 6008 and 6019 are aliphatic urethane triacrylates. PHOTOMER 6008 is a high viscosity acrylate resin. The viscosity range for PHOTOMER 6008 (neat) is about 12,000-20,000 cps at 60°C. PHOTOMER 6008 (homopolymer) exhibits the following properties: tensile strength of about 4800 psi and elongation percent of about 10. PHOTOMER 6019 is a medium viscosity acrylate resin. The viscosity range for PHOTOMER 6019 (neat) is about 2,500-4,000 cps at 60°C. PHOTOMER 6019 (UV-cured neat film) exhibits the following properties: tensile strength of about 8200 psi and elongation percent of about 8.

PHOTOMER 6210 is a low viscosity aliphatic urethane diacrylate. The viscosity range for PHOTOMER 6210 (neat) is about 8,500 to 15,000 cps at 25°C. PHOTOMER 6210 (with 33% tripropylene glycol diacrylate) exhibits the following properties: tensile strength of about 1400 psi and elongation percent of about 40.

BR-582 is an aliphatic linear polyether urethane acrylate. The viscosity of BR-582 (neat) is about 300,000 cps at 50°C. BR-582 (formulated with 30% isobornyl acrylate and Irgacure 184) exhibits the following properties: tensile strength of about 3300 psi and elongation percent of about 214.

Representative commercially available epoxy acrylate oligomers useful in the compositions of the present invention include PHOTOMER 3016 (available from Cognis, Corp.). PHOTOMER 3016 is bisphenol A epoxy diacrylate. The viscosity of PHOTOMER 3016 is about 4000 mPa.s at 60°C.

Representative commercially available metallic acrylate oligomers useful in the compositions of the present invention include CN 2400 series metallic acrylates (available from Sartomer Company, Inc., Exton, PA).

In accordance with the present invention, the acrylate oligomers may be employed in amounts of about 10% to about 70% by weight of the total composition, more desirably about 15% to about 50% by weight of the total composition.

### Reactive Diluents

The compositions of the present invention also may contain at least one reactive diluent. Reactive diluents may be selected from those materials known in the art, and may be straight-chained, branched or cyclic, and may be at least partially aliphatic. The reactive diluent may soften the acrylate composition, for example, to exhibit a Shore D hardness of at least about 85 once cured.

Desirable reactive diluents for use in the present invention include monomers such as alkyl acrylates, methacrylates or alkoxylated alkyl (meth)acrylates, having about 6-18 carbon atoms in the alkyl moiety of the molecule. A variety of reactive diluents may be used, such as, for example, ethoxylated bisphenol A dimethacrylate, propoxylated neopentyl glycol diacrylate, dipentaerythritol monohydroxy pentaacrylate ("DIPETA"), isobornyl acrylate and combinations thereof.

Representative commercially available ethoxylated bisphenol A dimethacrylate reactive diluents include SR348, SR9036, CD 540, CD 542, SR101, SR150 and SR 541, available from Sartomer Company, Inc., Exton, PA. Representative commercially available propoxylated neopentyl glycol diacrylate diluents include SR9003 and SR 9003IJ, available from Sartomer Company, Inc., Exton, PA. Representative commercially available DIPETA reactive diluents include SR399E, also available from Sartomer Company, Inc., Exton, PA.

Reactive diluents may be introduced independently in the overall composition or in a pre-mix of the urethane acrylate oligomer. In accordance with the present invention, reactive diluents may be employed in amounts of about 30% to about 90% by weight of the total composition, more desirably about 50% to about 85% by weight of the total composition.

Some embodiments of the present invention contain several reactive diluents, such as, for example, ethoxylated bisphenol A dimethacrylate in amounts of about 15-65%, propoxylated neopentyl glycol diacrylate in amounts of about 10-20%, dipentaerythritol monohydroxy pentaacrylate in amounts of about 1-10% and isobornyl acrylate in amounts of about 15-40% by weight of the total composition.

### Cure System

In applications where hearing aid components are to be made, the cure system is desirably one which is initiated by electromagnetic radiation. Photoradiation is desirable for its ability to produce a well-controlled cure and high quality parts efficiently. Various photoinitiators, such as UV, visible and infrared may be employed.

UV photoinitiators are generally effective in the 200 to 400 nm range, and particularly in the portion of the spectrum that borders on the invisible light and the visible portion just beyond this, e.g. >200 nm to about 390 nm.

A variety of UV photoinitiators may be employed. Photoinitiators, those that will respond to UV radiation to initiate and induce curing of the (meth)acryl functionalized curable component, which are useful in the present invention include benzophenone and substituted benzophenones, acetophenone and substituted acetophenones, benzoin and its alkyl esters, xanthone and substituted xanthones, diethoxy-acetophenone, benzoin methyl ether, benzoin ethyl ether, benzoin isopropyl ether, diethoxyxanthone, chloro-thio-xanthone, N-methyl diethanolamine-benzophenone, 2-hydroxy-2-methyl-1-phenyl-propan-1-one, 2-benzyl-2-(dimethylamino)-1-[4-(4-morpholinyl)phenyl]-1-butanone, bisacyl phosphine oxides and mixtures thereof. Photoinitiators suitable for use in the present invention that will respond to visible light to initiate and induce curing include camphoroquinone peroxyester initiators and 9-fluorene carboxylic acid peroxyesters. Thermal initiators include 2,2'-azobisisobutyronitrile. The initiators set forth above are for the purposes of illustration only and are in no way meant to limit the initiators that may be used in the present invention.

Examples of such UV initiators include, but are not limited to: methylbenzoylformate (commercially available as DAROCUR MBF from Ciba Specialty Chemicals Inc.), diphenyl (2,4,6-trimethylbenzoyl) phosphine oxide (commercially available as LUCIRIN TPO from BASF Corp.), 1-benzoyl cyclohexanol (commercially available as IRGACURE 184 from Ciba Specialty Chemicals Inc.), phenyl bis (2,4,6-trimethylbenzoyl) phosphine oxide (commercially available as IRGACURE 819 from Ciba Specialty Chemical, Inc.) and combinations thereof.

Photoinitiators may be employed in amounts of about 1% to about 10% by weight of the total composition. More desirably, the photoinitiator is present in amounts of about 2% to about 6% by weight of the total composition.

The cure system also may include stabilizers and inhibitors, as well as chelating agents to control and prevent premature peroxide decomposition and polymerization. Among those useful inhibitors include phenols such as hydroquinone and quinones. Chelating agents may be used to remove trace amounts of metal contaminants. An example of a useful chelating agent is the tetrasodium salt of ethylenediamine tetraacetic acid ("EDTA").

### Optional Additives

A variety of optional additives also may be included in the compositions of the present invention. For instance, agents such as antioxidants, thickeners, plasticizers, fillers, elastomers, thermoplastics, dispersion stabilizers, hindered amine light stabilizers, UV absorbers, additional monomers and other well-known additives may be incorporated where functionally desirable.

Example of suitable antioxidants for use in the compositions of the present invention include thiodiethylene bis (3,5-di-tert-butyl-4-hydroxyhydrocinnamate) (commercially available as IRGANOX 1035 from Ciba Specialty Chemicals Inc.), tetrakis-(methylene-(3,5-di-terbutyl-4-hydrocinnamate)methane (commercially available as IRGANOX 1010 from Ciba Specialty Chemicals Inc.) and combinations thereof. Antioxidants may be present in amounts of about 0.1% to about 1% by weight of the total composition, more desirably about 0.1% to about 0.5% by weight of the total composition.

An example of a suitable hindered amine light stabilizer ("HALS") is TINUVIN 292 (commercially available from Ciba Specialty Chemicals Inc.). Examples of suitable UV absorbers include TINUVIN 900 and TINUVIN 400 (both commercially available from Ciba Specialty Chemicals Inc.).

Additional monomers may include, for example, cyclic trimethylolpropane formal acrylate (commercially available as SR531 from Sartomer Company, Inc., Exton, PA), dimethylacrylamide, phenoxyethyl acrylate, alkoxylated hexanediol diacrylate (commercially available as SR564 from Sartomer Company, Inc., Exton, PA) and combinations thereof.

Various colorants also may be included in the inventive compositions. The term "colorants" is used to include dyes, pigments and other materials which can be used to impart color to the composition. Dyes are generally water-soluble organics, which often become water-insoluble once cured. Pigments may be organic or inorganic materials and are generally in the solid form.

Examples of useful pigments include, without limitation: white pigments, such as titanium oxide, zinc phosphate, zinc sulfide, zinc oxide and lithopone; red and red-orange pigments, such as iron oxide (maroon, red, light red), iron/chrome oxide, cadmium sulfoselenide and cadmium mercury (maroon, red, orange); ultramarine (blue, pink and violet), chrome-tin (pink) manganese (violet), cobalt (violet); orange, yellow and buff pigments such as barium titanate, cadmium sulfide (yellow), chrome (orange, yellow), molybdate (orange), zinc chromate (yellow), nickel titanate (yellow), iron oxide (yellow), nickel tungsten titanium, zinc ferrite and chrome titanate; brown pigments such as iron oxide (buff, brown), manganese/antimony/titanium oxide, manganese titanate, natural siennas (umbers), titanium tungsten manganese; blue-green pigments, such as chrome aluminate (blue), chrome cobolt-alumina (turquoise), iron blue (blue), manganese (blue), chrome and chrome oxide (green) and titanium green; as well as black pigments, such as iron oxide black and carbon black.

Combinations of pigments are generally used to achieve the desired color tone in the cured composition. Titanium and iron oxides, in combination, are particularly useful in creating flesh tones for hearing aids, and tips.

The colorants may be present in the compositions of the present invention in amounts sufficient to render the desired color. Colorants may be present, for example, in amounts of about 0.1 to about 10.0%, desirably about 0.2 to about 2.0% and more desirably in amounts of about 0.2 to about 0.6% by weight of the total composition. Insoluble pigments are the desired form of colorant useful in the compositions of the present invention.

Fillers may include inorganic fillers. At least a portion of the filler component may include filler particles in the 1-1,000 nanometer ("nm") range. The inorganic filler may be colloidal silica nanoparticle dispersions in acrylate resins, in amounts of up to about 50% by weight. A commercially available example of such filler particles is sold under the tradename NANOCRYL, such as NANOCRYL C 150, by Hans Chemie, Germany. NANOCRYL fillers are colloidal silica sols in unsaturated (meth)acrylates. In particular, NANOCRYL is a silica reinforced trifunctional acrylate monomer having a silica content of about 50% by weight. The silica component is surface-modified, synthetic SiO₂ nanospheres with a particle size of less than about 50 nm and a narrow particle size distribution. NANOCRYL is a colloidal dispersion of the silica nanoparticles in the (meth)acrylate.

Another commercially available example of filler particles is sold under the tradename NANOPOX, such as NANOPOX XP 22, by Hans Chemie, Germany. NANOPOX fillers are monodisperse silica filler dispersions in epoxy resins, at a level of up to about 50% by weight. NANOPOX fillers ordinarily are believed to have a particle size of about 5 nm to about 80 nm. And NANOPOX XP 22 is reported to contain 40 weight percent of silica particles having a particle size of about 15 to 20 nm in the diglycidyl ether of bisphenol-F epoxy resin.

### Tensile Properties

Standard measurements of the tensile properties of various substances are currently performed using tensile test procedures, such as those set forth in ASTM D882 herein incorporated by reference. These tensile test procedures are used for determining tensile properties of plastics in the form of thin sheeting, including film. These test methods measure the force required to break a specimen and the extent to which the material stretches or elongates to that breaking point. A stress to strain diagram is produced from these tests, which is then used to determine tensile modulus.

Tensile property measurements use a constant rate-of-grip separation machine, in which the test specimen is held between two grips (one fixed and one movable) in accordance with ASTM D882. A drive member separates the two grips at a controlled velocity. The force required to elongate the test specimen is measured (with the corresponding amount of material that has elongated) until the specimen breaks. A stress-strain curve may be produced, from which modulus of elasticity is calculated. The modulus of elasticity is the ratio between the stress per unit area to the amount of deformation resulting from that stress. A high tensile modulus generally means that the material is more rigid, i.e., more stress is necessary to produce a given amount of strain.

The curable compositions of the present invention, for example, may have a tensile modulus of at least about 300,000 psi (pounds per square inch).

### Flexural Properties

Standard measurement of the flexural properties of various substances are currently performed using flexural test procedures, such as those set forth in ASTM D790 ("Flexural Properties of Un-Reinforced and Reinforced Plastics") herein incorporated by reference.

Flexural modulus provides an indication of the stiffness of cured non-elastomeric adhesive materials. More specifically, the test procedures may measure the force required to bend a material under three-point loading conditions.

The curable compositions of the present invention, for example, may have a flexural modulus of at least about 300,000 psi (pounds per square inch).

### Hardness

Standard measurements of the hardness of various substances are currently performed using durometer hardness test procedures, such as those set forth in ASTM D2240 herein incorporated by reference. The durometer hardness procedures are used for determining indentation hardness of various substances, and are particularly useful for elastomeric materials. These test methods measure the depression force of a specific type of indentor as it is forced under specific conditions against the material's surface. Due to the various parameters which influence hardness determinations, different durometer scales have been established. A particular scale is chosen depending on the type of material to be measured. For example, materials which are relatively soft, such as elastomeric materials, are measured on a Shore A scale. Slightly harder materials are measured on a Shore D scale. Shore D scale measurements use a steel rod indentor shaped with a pointed end, and a calibrated spring force, as shown in Figure 1 and Table 1, respectively of ASTM D2240. The indentor descends at a controlled rate against the specimen surface and a reading is recorded within a specified time period. This procedure is repeated multiple times at different positions on the specimen and the arithmetic mean of the results yields the Shore D measurement.

Durometer scales which are used for durometer hardness measurements include Shore A, B, C, D, DO, O, OO, and M. Each of theses scales has units from 0 to 100. There is no overlap between the scales, although certain materials may be suitable for testing on both scales. The geometry of the indentor and calibrated spring force scales influence the measurements, such that no simple relationship exists between the measurements obtained between different types of durometers. For example, the test for Shore D, which is designed for harder materials, is distinct from Shore A in that the indentor is shaped with a pointed tip and the calibrated spring force has a higher force scale then Shore A.

The curable compositions of the present invention, for example, may have a Shore D hardness of at least about 85 once cured.

### Process of Making Hearing Aid Components

The present invention is directed to methods of making hearing aid components. The hearing aid component may include a hearing aid housing and a hearing aid tip, which may be mated to the hearing aid housing. In some embodiments, the hearing aid component may be a single unit forming both the hearing aid housing and tip.

In accordance with the present invention, SFF techniques, particularly stereolithography and Inkjet processes, are employed to make the hearing aid components. Stereolithograpy uses an additive, built-up process to form a three-dimensional article, which is commonly referred to as rapid prototyping. In particular, stereolithography uses photo-radiation to cure additive layers of a composition to form a polymeric article, e.g., a hearing aid component.

More specifically, stereolithography uses data of the three-dimensional shape of the area of application in an individual's ear to control the layer by layer process of forming the article. In accordance with the present invention, data of the three-dimensional size and shape of a region in an individual's ear, which corresponds to the hearing aid component being made, may first be provided. The three-dimensional data may be provided by obtaining digital representations of the region in the individual's ear. The region may be, for example, the outer ear, ear canal or a combination thereof.

According to the present invention, a curable composition may be prepared by combining at least one acrylate oligomer, at least one reactive diluent and a cure system including a photoinitiator. The acrylate oligomer may be a urethane acrylate, epoxy acrylate, metallic acrylate or any combination thereof. The reactive diluent may be selected from ethoxylated bisphenol A dimethacrylate, propoxylated neopentyl glycol diacrylate, dipentaerythritol monohydroxy pentaacrylate and combinations thereof.

Once the components are combined, the curable composition may be exposed to photo-radiation for a time and intensity sufficient to solidify (cure) a layer at the surface of the composition. A UV laser may be used for curing. Desirably, this layer corresponds to a cross-section of the hearing aid component being made. This may be achieved by controlling the UV laser with the digitized three-dimensional data of the region in the individual's ear. Once the first layer is cured, a new layer of the curable composition may be applied to the first cured layer and similarly cured by photo-radiation. The new layer may be applied by lowering the first cured layer into the curable composition and covering it with a new layer of the composition. Layers may be repeatedly added and solidified in this manner until the hearing aid component having the desired three-dimensional shape is formed.

Desirably, each layer of the curable composition is cured to produce one or more of the following properties: Shore D hardness of at least about 85; tensile modulus of at least about 300,000 psi; and flexural modulus of at least about 300,000 psi.

Stereolithography techniques are described in detail in U.S. Patent Nos. 6,540,045, 6,533,062, 6,413,697, and 6,136,497, each of which is incorporated herein by reference.

In some embodiments, Inkjet processes may be used to make the hearing aid component. Inkjet processes include both thermal phase change systems and photopolymer phase change systems. In thermal phase change systems, the polymer composition is first melted by heating. The molten polymer composition is jetted from one or more nozzles and the composition solidifies (cures) upon impact. The nozzles may be controlled by a computer program, which prescribes the configuration of each layer forming the three-dimensional object. The process is continued to add layers of the composition until the final three-dimensional object, i.e., hearing aid component, is formed. Thermal phase change techniques are described in detail in U.S. Patent Nos. 6,132,665, 6,406,531, 6,133,353, 6,395,811, 6,528,613 and 6,476,122, each of which is incorporated herein by reference.

In photopolymer phase change systems, the photopolymer composition is ejected from inkjet heads and cured with UV flood lamps. The inkjet heads may be controlled by a computer program, which prescribes the configuration of each layer forming the three-dimensional object. The process is continued to add and cure layers of the composition until the final three-dimensional object, i.e., hearing aid component, is formed. Photopolymer phase change techniques are described in detail in U.S. Patent Nos. 6,534,128, 6,467,897 and 6,259,962 and U.S. Patent Application Publication No. 2003/0032692, each of which is incorporated herein by reference.

The above-described SFF processes may be used to form a hearing aid housing, hearing aid tip or combination thereof. The hearing aid housing and tip may be formed separately and mated together. Alternatively, the hearing aid component may be a single unit including the housing and tip.

In some embodiments, the above-described processes may be used to prepare a hearing aid housing. The hearing aid housing may be mated to a tip component to form a hearing aid assembly. The tip component may be prepared in accordance with the curable composition and process described herein, or alternatively, the tip may be prepared in accordance with the disclosure of U.S. Patent No. 6,829,362 or U.S. Patent Application No. 10/922,458, both referred to above.

For instance, a method of making the tip component may include pouring the curable composition disclosed in U.S. Patent No. 6,829,362 or U.S. Patent Application No. 10/922,458 into the lower portion of a mold cavity, which is the tip cavity, of a light-penetrable mold, the mold having an exposed, generally upward-facing surface. The composition covers a major amount of the generally upward-facing surface of the tip cavity of the mold and fills the majority of the tip cavity. The surface is exposed to ultra-violet radiation through the transparent mold surface for a time and intensity sufficient to cure the composition. Once the tip is cured, it may be mated or adhered to the hearing aid housing by crosslinkage.

In accordance with the methods of the present invention, the resulting hearing aid components are self-supporting, free from surface blemishes and uniform in color. They can be pigmented to match a variety of skin tones and are ideally suited for hearing aid components, such as hearing aid housings and tips.

The following examples are intended to be non-limiting illustrations of compositions of the present invention.

### EXAMPLES

### Example 1:

A curable composition was prepared in accordance with the present invention and tested for various physical properties. Table 1 below lists the weight percents for each component contained in the curable composition.

**TABLE 1**

| COMPONENT | WEIGHT % |
|---|---|
| Ethoxylated Bisphenol A Dimethacrylate | 59.75 |
| Propoxylated Neopentyl Glycol Diacrylate | 17.00 |
| Aliphatic Urethane Diacrylate | 15.00 |
| Dipentaerythritol Monohydroxy Pentaacrylate | 5.00 |
| Methylbenzoylformate | 3.00 |
| Diphenyl (2,4,6-Trimethylbenzoyl) Phosphine Oxide | 0.25 |
| Antioxidant¹ | 0.20 |

| | |
|---|---|
| ¹ Thiodiethylene bis (3,5-di-tert-butyl-4-hydroxyhydrocinnamate) (IRGANOX 1035) | |

Once cured, the composition was tested for: yellowness index (determined in accordance with ASTM E313); tensile properties (determined in accordance with ASTM D882); flexural properties (determined in accordance with ASTM D790); hardness (determined in accordance with ASTM D2240); and shrinkage. The results of the tests with respect to each property are provided in Table 2 below.

**TABLE 2**

| PHYSICAL PROPERTY | RESULTS |
|---|---|
| Yellowness | |
| Initial | 11.42 |
| Post-cure | 11.00 |
| Delta | 0.42 |
| | |
| Tensile Properties | |
| Modulus (psi) | 315,000 |
| Stress at break (psi) | 7,400 |
| Strain at break (%) | 4.0 |
| | |
| Flexural Properties | |
| Modulus (psi) | 346,000 |
| Stress at max load (psi) | 10,800 |
| Strain at max load (in/in) | 0.05 |
| | |
| Hardness (Shore D) | 85 |
| | |
| Shrinkage | |
| Volume (%) | 7.3 |
| Linear (%) | 2.5 |

### Examples 2-17:

Curable compositions were prepared in accordance with the present invention and tested for various physical properties. Tables 3-5 depict the weight percents for each component contained in the curable compositions of Examples 2-17.

**TABLE 3**

| COMPONENT | EXAMPLE NO. (WEIGHT %) | | | | |
|---|---|---|---|---|---|
| | 2 | 3 | 4 | 5 | 6 |
| Aliphatic urethane triacrylate¹ | | | | | 15.00 |
| Acrylated urethane² | | 15.00 | 15.00 | 15.00 | |
| Aliphatic urethane triacrylate¹ (50%), Triethylene glycol dimethacrylate (25%), Hydroxypropyl methacrylate & Hydroxyethyl methacrylate (11%) | 97.75 | | | | |
| Ethoxylated Bisphenol A Dimethacrylate | | 59.50 | 59.75 | 57.55 | 57.55 |
| Dipentaerythritol Monohydroxy Pentaacrylate | | 5.00 | 5.00 | 5.00 | 5.00 |
| Neopentylglycol Propoxylate Diacrylate | | 17.00 | 17.00 | 17.00 | 17.00 |
| 1-Benzoyl Cyclohexanol³ | 2.00 | 3.00 | 3.00 | | |
| Diphenyl (2,4,6-Trimethylbenzoyl) Phosphine Oxide | 0.25 | 0.50 | 0.25 | 0.25 | 0.25 |
| Thioethylene bis-(3,5-di-tert-butyl-4-hydroxy) hydrocinnamate | | | | 0.20 | 0.20 |
| Methylbenzoylformate | | | | 5.00 | 5.00 |

| | | | | | |
|---|---|---|---|---|---|
| ¹ PHOTOMER 6008 (available from Cognis, Corp.) ² RSX 89462 (available from UCB Group) ³ IRGACURE 184 (available from Ciba Specialty Chemicals) | | | | | |

**TABLE 4**

| COMPONENT | EXAMPLE NO. (WEIGHT %) | | | |
|---|---|---|---|---|
| | 7 | 8 | 9 | 10 |
| Bisphenol A Epoxy Diacrylate¹ | 25.00 | 25.00 | 25.00 | 20.00 |
| Metallic Acrylate Oligomer²+Isobornyl acrylate | 40.00 | | | |
| Metallic Acrylate Oligomer² | | 40.00 | | |
| Metallic Acrylate Oligomer² | | | 40.00 | |
| Metallic Acrylate Oligomer² | | | | 30.00 |
| Neopentylglycol Propoxylate Diacrylate | 32.75 | 32.75 | 32.75 | 47.75 |
| 1-Benzoyl Cyclohexanol³ | 2.00 | 2.00 | 2.00 | 2.00 |
| Diphenyl (2,4,6-Trimethylbenzoyl) Phosphine Oxide | 0.25 | 0.25 | 0.25 | 0.25 |

| | | | | |
|---|---|---|---|---|
| ¹ PHOTOMER 3016 (available from Cognis, Corp.) ² CN 2402 (available from Sartomer) ³ IRGACURE 184 (available from Ciba Specialty Chemicals) | | | | |

**TABLE 5**

| COMPONENT | EXAMPLE (WEIGHT %) | | | | | | |
|---|---|---|---|---|---|---|---|
| | 11 | 12 | 13 | 14 | 15 | 16 | 17 |
| Acrylated urethane¹ | | | | 40.00 | | | |
| Aliphatic Urethane Oligomer | | | 20.00 | | | | |
| Bisphenol A Epoxy Diacrylate² | 20.00 | | 20.00 | 25.00 | 20.00 | 20.00 | 25.00 |
| Metallic Acrylate Oligomer³ +Isobornyl acrylate | | | | | | | 40.00 |
| Aliphatic Urethane Oligomer and Cyclic Trimethylolpropane Formal Acrylate (25%)⁴ | 35.00 | 40.00 | | | 35.00 | 35.00 | |
| Ethoxylated Bisphenol A Dimethacrylate | | 25.00 | | | | | |
| Neopentylglycol Propoxylate Diacrylate | 42.75 | 32.75 | 57.75 | 32.75 | 32.75 | 32.75 | 32.75 |
| Cyclic Trimethylolpropane Formal Acrylate | | | | | 10.00 | 10.00 | |
| 1-Benzoyl Cyclohexanol⁵ | 2.00 | 2.00 | 2.00 | 2.00 | 2.00 | 2.00 | 2.00 |
| Diphenyl (2,4,6-Trimethylbenzoyl) Phosphine Oxide | 0.25 | 0.25 | 0.25 | 0.25 | 0.25 | 0.25 | 0.25 |
| Hindered Amine Light Stabilizer⁶ | | | | | | 1.00 | 1.00 |
| Hydroxybenzotriazole⁷ | | | | | | 1.00 | 1.00 |

| | | | | | | | |
|---|---|---|---|---|---|---|---|
| ¹ RSX 89462 (available from UCB Group) ² PHOTOMER 3016 (available from Cognis, Corp.) ³ CN 2402 (available from Sartomer) ⁴ PRO-7157 (available from Sartomer Company, Inc.) ⁵IRGACURE 184 (available from Ciba Specialty Chemicals) ⁶ TINUVIN 292 (available from Ciba Specialty Chemicals) ⁷ TINUVIN 900 (available from Ciba Specialty Chemicals) | | | | | | | |

Once cured, compositions 2-17 were tested for: yellowness index (determined in accordance with ASTM E313); tensile properties (determined in accordance with ASTM D882); flexural properties (determined in accordance with ASTM D790); and/or hardness (determined in accordance with ASTM D2240). The results of the tests with respect to each property for the indicated compositions are provided in Tables 6-9 below.

**TABLE 6**

| TENSILE PROPERTIES | EXAMPLE NO. | | |
|---|---|---|---|
| | 2 | 3 | 4 |
| Modulus (PSI) | 301,000 | 317,000 | 297,000 |
| Stress @ Max Load (PSI) | 7,690 | 7,430 | 5,970 |
| Strain @ Max Load (%) | 5 | 4 | 3 |
| Stress @ Break (PSI) | 6,360 | 7,430 | 5,970 |

**TABLE 7**

| FLEXURAL PROPERTIES | EXAMPLE NO. | | | |
|---|---|---|---|---|
| | 2 | 4 | 5 | 6 |
| Modulus (PSI) | 326,000 | 346,000 | 270,000 | 320,000 |
| Stress @ Max Load (PSI) | 11,370 | 10,820 | 7,960 | 10,250 |
| Strain @ Max Load (%) | 0.05 | 0.04 | 0.04 | 0.05 |

**TABLE 8**

| HARDNESS | EXAMPLE NO. | | | | |
|---|---|---|---|---|---|
| | 2 | 3 | 4 | 5 | 6 |
| Shore D | 83 | 85 | 84 | 83 | 82 |

**TABLE 9**

| YELLOWNESS | EXAMPLE NO. | | | | | |
|---|---|---|---|---|---|---|
| | 4 | 5 | 7 | 15 | 16 | 17 |
| Initial | 9.25 | 12.28 | 7.37 | 4.84 | 6.65 | 8.07 |
| 120" Post Cure | 18.22 | 11.58 | 13.53 | 7.75 | 7.18 | 9.88 |
| Delta | 8.97 | -0.70 | 6.16 | 2.91 | 0.54 | 1.81 |

### Examples 18-34:

Curable compositions were prepared in accordance with the present invention and tested for cure through depth. Tables 10-11 depict the weight percents for each component contained in the curable compositions of Examples 18-34. The cure through depth of the compositions was measured by exposure to a 550 mW/cm² UV lamp for 3, 7 and 10 second intervals. These results also are provided in Tables 10-11.

**TABLE 10**

| COMPONENT | EXAMPLE NO. (WEIGHT %) | | | | | | | |
|---|---|---|---|---|---|---|---|---|
| | 18 | 19 | 20 | 21 | 22 | 23 | 24 | 25 |
| Bisphenol A Epoxy Diacrylate¹ | 20.00 | 20.00 | 20.00 | 20.00 | 20.00 | 20.00 | 20.00 | 30.00 |
| Aliphatic urethane triacrylate² | 20.00 | 20.00 | 20.00 | 20.00 | 20.00 | | | |
| 50% Nanosilica and 50% Trimethylol Propane Triacrylate³ | 50.00 | 50.00 | 50.00 | 50.00 | 50.00 | | 30.00 | |
| Aliphatic Urethane Oligomer and Cyclic Trimethylolpropane Formal Acrylate (25%)⁴ | | | | | | 35.00 | | |
| Aliphatic Urethane Oligomer⁵ | | | | | | | 20.00 | |
| Aliphatic Urethane Oligomer⁶ | | | | | | | | 37.60 |
| Neopentylglycol Propoxylate Diacrylate | | | | | | 42.60 | | |
| Dimethylacrylamide | 8.50 | 9.50 | 8.60 | 8.60 | 7.60 | | | |
| Phenoxyethylacrylate | | | | | | | 27.60 | |
| Cyclic Trimethylolpropane Formal Acrylate⁷ | | | | | | | | 10.00 |
| Alkoxylated Hexanediol Diacrylate⁸ | | | | | | | | 20.00 |
| Camphorquinone | 0.50 | | | 0.50 | 0.50 | 0.50 | 0.50 | 0.50 |
| N,N-dimethyl-p-toluidine ("DMpT") | 0.50 | | | 0.50 | 0.50 | 0.50 | 0.50 | 0.50 |
| Bisacyl Phosphine Oxide | 0.10 | 0.10 | 1.00 | | 1.00 | 1.00 | 1.00 | 1.00 |
| Pigment | 0.40 | 0.40 | 0.40 | 0.40 | 0.40 | 0.40 | 0.40 | 0.40 |
| | | | | | | | | |
| CURE THROUGH DEPTH (mm) | | | | | | | | |
| 3 second cure | 0.00 | 0.00 | 0.00 | 0.00 | 0.24 | 0.00 | 0.35 | 0.00 |
| 7 second cure | 0.45 | 0.00 | 0.24 | 0.35 | 0.61 | 0.53 | 0.55 | 0.39 |
| 10 second cure | 0.59 | 0.00 | 0.32 | 0.48 | 0.68 | 0.64 | 0.74 | 0.61 |

| | | | | | | | | |
|---|---|---|---|---|---|---|---|---|
| ¹ PHOTOMER 3016 (available from Cognis, Corp.) ² PHOTOMER 6008 (available from Cognis, Corp.) ³ NANOCRYL C-150 (available from Hanse Chemie) ⁴ PRO 7157 (available from Sartomer Company, Inc.) ⁵ BR-582E (available from Bomar Specialties Co.) ⁶ PHOTOMER 6019 (available from Cognis, Corp.) ⁷ SR531 (available from Sartomer Company, Inc.) ⁸ SR564 (available from Sartomer Company, Inc.) | | | | | | | | |

**TABLE 11**

| COMPONENT | EXAMPLE NO. (WEIGHT %) | | | | | | | | |
|---|---|---|---|---|---|---|---|---|---|
| | 26 | 27 | 28 | 29 | 30 | 31 | 32 | 33 | 34 |
| Bisphenol A Epoxy Diacrylate¹ | | | | 15.00 | | 15.00 | 15.00 | 12.00 | 12.00 |
| Aliphatic urethane triacrylate² | | 15.00 | 27.60 | | 15.00 | | | | |
| Acrylated urethane³ | 50.00 | | | | | | | | |
| 50% Nanosilica and 50% Trimethylol Propane Triacrylate⁴ | 12.60 | 17.60 | | | 17.60 | | | | |
| Aliphatic Urethane Oligomer | | 30.00 | 15.00 | 30.00 | 30.00 | 30.00 | 30.00 | 25.00 | 25.00 |
| Isobornyl Acrylate | 30.00 | 35.00 | 35.00 | 35.00 | 35.00 | 35.00 | 45.00 | 40.00 | 42.00 |
| Dipentaerythritol Monohydroxy Pentaacrylate | 5.00 | | 5.00 | 5.00 | | 5.00 | 5.00 | 5.00 | 5.00 |
| Neopentylglycol Propoxylate Diacrylate | | | 15.00 | 12.60 | | 12.60 | 12.60 | 13.60 | 13.60 |
| Camphorquinone | 0.50 | 0.50 | 0.50 | 0.50 | | | | | |
| DMpT | 0.50 | 0.50 | 0.50 | 0.50 | | | | | |
| Bisacyl Phosphine Oxide | 1.00 | 1.00 | 1.00 | 1.00 | 2.00 | 2.00 | 2.00 | 4.00 | 2.00 |
| Pigment | 0.40 | 0.40 | 0.40 | 0.40 | 0.40 | 0.40 | 0.40 | 0.40 | 0.40 |
| | | | | | | | | | |
| CURE THROUGH DEPTH (mm) | | | | | | | | | |
| 3 second cure | 0.00 | 0.00 | 0.00 | 0.22 | 0.15 | 0.15 | | | 0.00 |
| 7 second cure | 0.54 | 0.60 | 0.45 | 0.54 | 0.39 | 0.43 | 0.32 | | 0.33 |
| 10 second cure | 0.68 | 0.80 | 0.80 | 0.72 | 0.50 | 0.54 | 0.45 | | 0.45 |

| | | | | | | | | | |
|---|---|---|---|---|---|---|---|---|---|
| ¹ PHOTOMER 3016 (available from Cognis, Corp.) ² PHOTOMER 6008 (available from Cognis, Corp.) ³ RSX 89462 (available from UCB Group) ⁴ NANOCRYL C-150 (available from Hanse Chemie) | | | | | | | | | |

### Examples 35-37:

Curable compositions were prepared in accordance with the present invention. Table 12 depicts the weight percents for each component contained in the curable compositions of Examples 35-37.

**TABLE 12**

| COMPONENT | EXAMPLE NO. (WEIGHT %) | | |
|---|---|---|---|
| | 35 | 36 | 37 |
| Aliphatic Urethane Oligomer and Cyclic Trimethylolpropane Formal Acrylate (25%) | 29.00 | 35.00 | 40.00 |
| Bisphenol A Epoxy Diacrylate | 11.00 | 17.00 | |
| Ethoxylated Bisphenol A Dimethacrylate | | | 19.00 |
| Isobornyl Acrylate | 37.00 | 25.00 | 18.00 |
| Dipentaerythritol Monohydroxy Pentaacrylate | 5.00 | 5.00 | 5.00 |
| Neopentylglycol Propoxylate Diacrylate | 14.025 | 14.025 | 14.025 |
| 1-benzoyl cyclohexanol¹ | 3.00 | 3.00 | 3.00 |
| Phenyl bis (2,4,6-trimethylbenzoyl) phosphine oxide² | 0.50 | 0.50 | 0.50 |
| Hindered Amine Light Stabilizer³ | 0.10 | 0.10 | 0.10 |
| UV Absorber⁴ | 0.10 | 0.10 | 0.10 |
| Pigment | 0.275 | 0.275 | 0.275 |

| | | | |
|---|---|---|---|
| ¹ IRGACURE 184 (available from Ciba Specialty Chemicals) ² IRGACURE 819 (available from Ciba Specialty Chemicals) ³ TINUVIN 292 (available from Ciba Specialty Chemicals) ⁴ TINUVIN 400 (available from Ciba Specialty Chemicals) | | | |

## Claims

1. A method of making a hearing aid component comprising the steps of:
(a) providing data of the three-dimensional size and shape of a region in an individual's ear, wherein the region corresponds to the hearing aid component to be made;
(b) providing a first amount of a curable composition comprising:
(i) at least one acrylate oligomer selected from the group consisting of urethane acrylate oligomers, epoxy acrylate oligomers, metallic acrylate oligomers and combinations thereof;
(ii) at least one reactive diluent selected from the group consisting of ethoxylated bisphenol A dimethacrylate, propoxylated neopentyl glycol diacrylate, dipentaerythritol monohydroxy pentaacrylate, isobornyl acrylate and combinations thereof; and
(iii) a cure system comprising at least one photoinitiator;
(c) exposing the composition to photo-radiation for a time and intensity sufficient to cure a layer on the surface of the composition which corresponds to a cross-section of the hearing aid component;
(d) providing a second amount of a curable composition;
(e) applying the second amount to the cured layer to form a new layer and exposing the new layer to photo-radiation for a time and intensity sufficient to cure the new layer; and
(f) repeating steps (d) and (e) until the hearing aid component is formed.

2. The method of claim 1, wherein each layer of the curable composition is cured to produce one or more properties selected from the group consisting of: Shore D hardness of at least about 85; tensile modulus of at least about 300,000 psi; and flexural modulus of at least about 300,000 psi.

3. The method of claim 1, wherein the step of providing data of the three-dimensional size and shape of a region in an individual's ear comprises obtaining one or more digital representations of the region in the individual's ear.

4. The method of claim 1, wherein the step of exposing the composition to photo-radiation comprises exposing the composition to a UV laser which is controlled by the three-dimensional data of the region in the individual's ear.

5. The method of claim 1, wherein the region in the individual's ear is selected from the group consisting of: the outer ear; the ear canal; and combinations thereof.

6. The method of claim 1, wherein the step of applying a second amount of a curable composition to the cured layer comprises lowering the cured layer into the composition and covering the cured layer with a new layer.

7. The method of claim 1, wherein the hearing aid component comprises a hearing aid housing.

8. The method of claim 1, wherein the hearing aid component comprises a hearing aid tip.

## Patentansprüche

1. Ein Verfahren zur Herstellung einer Hörgerätkomponente, umfassend die Schritte:
(a) Bereitstellen von Daten der dreidimensionalen Größe und Form eines Bereichs im Ohr einer Person, wobei der Bereich der herzustellenden Hörgerätkomponente entspricht,
(b) Bereitstellen einer ersten Menge einer härtbaren Zusammensetzung, umfassend:
(i) wenigstens ein Acrylatoligomer, ausgewählt aus der Gruppe, bestehend aus Urethanacrylatoligomeren, Epoxidacrylatoligomeren, metallischen Arcrylatoligomeren und Kombinationen davon,
(ii) wenigstens ein reaktives Verdünnungsmittel, ausgewählt aus der Gruppe, bestehend aus ethoxyliertem Bisphenol-A-Dimethacrylat, propoxyliertem Neopentylglycoldiacrylat, Dipentaerythritmonohydroxypentaacrylat, Isobornylacrylat und Kombinationen davon, und
(iii) ein Härtungssystem, umfassend wenigstens einen Photoinitiator,
(c) Einwirkenlassen von Lichtstrahlung auf die Zusammensetzung für einen Zeitraum und mit einer Intensität, ausreichend, um eine Schicht auf der Oberfläche der Zusammensetzung zu härten, die einem Querschnitt der Hörgerätkomponente entspricht,
(d) Bereitstellen einer zweiten Menge einer härtbaren Zusammensetzung,
(e) Aufbringen der zweiten Menge auf die gehärtete Schicht, um eine neue Schicht zu bilden, und Einwirkenlassen von Lichtstrahlung auf die neue Schicht für einen Zeitraum und mit einer Intensität, ausreichend, um die neue Schicht zu härten, und
(f) Wiederholen der Schritte (d) und (e), bis die Hörgerätkomponente gebildet ist.

2. Das Verfahren gemäß Anspruch 1, wobei jede Schicht der härtbaren Zusammensetzung gehärtet wird, um eine oder mehrere Eigenschaften zu erzeugen, ausgewählt aus der Gruppe, bestehend aus: Shore-D-Härte von wenigstens etwa 85; Zugmodul von wenigstens etwa 300 000 psi und Biegemodul von wenigstens etwa 300 000 psi.

3. Das Verfahren gemäß Anspruch 1, wobei der Schritt des Bereitstellens von Daten der dreidimensionalen Größe und Form eines Bereichs im Ohr einer Person die Aufnahme einer oder mehrerer digitaler Darstellungen des Bereichs im Ohr der Person umfasst.

4. Das Verfahren gemäß Anspruch 1, wobei der Schritt des Einwirkenlassens von Lichtstrahlung auf die Zusammensetzung das Einwirkenlassen eines UV-Lasers, der durch die dreidimensionalen Daten des Bereichs im Ohr der Person gesteuert wird, auf die Zusammensetzung umfasst.

5. Das Verfahren gemäß Anspruch 1, wobei der Bereich im Ohr der Person ausgewählt ist aus der Gruppe, bestehend aus dem Außenohr, dem Gehörgang und Kombinationen davon.

6. Das Verfahren gemäß Anspruch 1, wobei der Schritt des Aufbringens einer zweiten Menge einer härtbaren Zusammensetzung auf die gehärtete Schicht das Absenken der gehärteten Schicht in die Zusammensetzung und das Überziehen der gehärteten Schicht mit einer neuen Schicht umfasst

7. Das Verfahren gemäß Anspruch 1, wobei die Hörgerätkomponente ein Hörgerätgehäuse umfasst.

8. Das Verfahren gemäß Anspruch 1, wobei die Hörgerätkomponente eine Hörgerätspitze umfasst.

## Revendications

1. Procédé de fabrication d'un composant de prothèse auditive comprenant les étapes suivantes :
(a) fournir des données sur la taille et la forme tridimensionnelles d'une région d'une oreille d'un individu, la région correspondant au composant de prothèse auditive à fabriquer ;
(b) prévoir une première quantité d'une composition durcissable comprenant :
(i) au moins un oligomère acrylate choisi dans le groupe comprenant des oligomères uréthane-acrylate, des oligomères époxy-acrylate, des oligomères acrylate métalliques, et leurs combinaisons ;
(ii) au moins un diluant réactif choisi dans le groupe comprenant le diméthacrylate de bisphénol A éthoxylé, le diacrylate de néopentyl glycol propoxylé, le monohydroxy-pentaacrylate de dipentaérythritol, l'acrylate d'isobornyle, et leurs combinaisons ; et
(iii) un système durcisseur comprenant au moins un photoinitiateur ;
(c) exposer la composition à une photoradiation pendant une durée et à une intensité suffisantes pour durcir une couche en surface de la composition, qui correspond à une section transversale du composant de prothèse auditive ;
(d) prévoir une seconde quantité d'une composition durcissable ;
(e) appliquer la seconde quantité sur la couche durcie pour former une nouvelle couche et exposer la nouvelle couche à la photoradiation pendant une durée et à une intensité suffisantes pour durcir la nouvelle couche ; et
(f) répéter les étapes (d) et (e) jusqu'à ce que le composant de prothèse auditive soit formé.

2. Procédé selon la revendication 1, dans lequel chaque couche de la composition durcissable est durcie pour produire une ou plusieurs propriétés choisies dans le groupe comprenant : dureté Shore D d'au moins environ 85 ; module de traction d'au moins environ 300 000 psi ; et module de flexion d'au moins environ 300 000 psi.

3. Procédé selon la revendication 1, dans lequel l'étape de mise à disposition de données sur la taille et la forme tridimensionnelles d'une région d'une oreille d'un individu consiste à obtenir une ou plusieurs représentations numériques de la région de l'oreille d'un individu.

4. Procédé selon la revendication 1, dans lequel l'étape d'exposition de la composition à la photoradiation consiste à exposer la composition à un laser UV qui est commandé par les données tridimensionnelles de la région de l'oreille d'un individu.

5. Procédé selon la revendication 1, dans lequel la région de l'oreille d'un individu est choisie dans le groupe comprenant l'oreille externe, le canal auditif et leurs combinaisons.

6. Procédé selon la revendication 1, dans lequel l'étape d'application d'une seconde quantité d'une composition durcissable sur la couche durcie consiste à abaisser la couche durcie dans la composition et recouvrir la couche durcie d'une nouvelle couche.

7. Procédé selon la revendication 1, dans lequel le composant de prothèse auditive comprend un logement de prothèse auditive.

8. Procédé selon la revendication 1, dans lequel le composant de prothèse auditive comprend une tête de prothèse auditive.
